# EUROPÄISCHE PATENTSCHRIFT

(11) **EP 2 890 307 B1**
(45) Veröffentlichungstag und Bekanntmachung des Hinweises auf die Patenterteilung: **17.03.2021**
(21) Anmeldenummer: 13750714.1
(22) Anmeldetag: 21.08.2013
(51) Int. Cl.: A61B 17/11, A61B 18/14

(54) **ELEKTROCHIRURGISCHES INSTRUMENT ZUR HERSTELLUNG EINER END-ZU-END-ANASTOMOSE**
ELECTROSURGICAL INSTRUMENT FOR PRODUCING AN END-TO-END ANASTOMOSIS
INSTRUMENT ÉLECTROCHIRURGICAL PERMETTANT DE PRODUIRE UNE ANASTOMOSE BOUT À BOUT

(30) Priorität: 28.08.2012 DE 102012107919
(43) Veröffentlichungstag der Anmeldung: 08.07.2015
(73) Patentinhaber: Aesculap AG, 78532 Tuttlingen (DE)
(72) Erfinder: WEISSHAUPT, Dieter, 78194 Immendingen (DE); MORALES, Pedro, 78532 Tuttlingen (DE); ROTHWEILER, Christoph, 78166 Donaueschingen (DE)
(74) Vertreter: Winter, Brandl, Fürniss, Hübner, Röss, Kaiser, Polte - Partnerschaft mbB
(86) Internationale Anmeldenummer: PCT/EP2013/067394
(87) Internationale Veröffentlichungsnummer: WO 2014/033032

(56) Entgegenhaltungen:
- WO-A1-99/65409
- US-A1- 2007 276 363
- US-A1- 2010 100 122

## Beschreibung

Die vorliegende Erfindung betrifft ein elektrochirurgisches Instrument zur Herstellung einer End-zu-End-Anastomose zwischen zwei Hohlorgan- oder Gefäßabschnitten gemäß anhängigem Anspruch 1. Weitere bevorzugte Ausführungsformen sind in den abhängigen Ansprüchen aufgeführt.

Um nach einem chirurgischen Eingriff zwei Hohlorganabschnitte, z.B. zwei Darmabschnitte, an ihren offenen Enden wieder miteinander zu verbinden, d.h. zur Herstellung einer End-zu-End-Anastomose, werden verschiedene Instrumente und Verfahren eingesetzt. End-zu-End-Anastomose können durch Nähen, durch Klammernahtgeräte, insbesondere Zirkularstapler, oder durch TFT (Tissue Fusion Technology bzw. Gewebefusionstechnik) - Instrumente hergestellt werden. Speziell bei Darmanastomosen wird routinemäßig ein Zirkularstapler eingesetzt. Bei Anastomosen im Bereich des Dickdarms wird das Instrument transanal eingeführt, so dass es sich im Inneren des Darms befindet. Nach erfolgter Anastomose wird das Instrument durch den Anus wieder entnommen. Diese Vorgehensweise ist bei Anastomosen des Dünndarms nicht möglich, da der Weg zur natürlichen Körperöffnung zu lang ist. Die Operation erfolgt durch die Bauchdecke, wobei kurz vor dem einen Ende der beiden zu verbindenden Darmabschnitte ein seitlicher Schnitt vorgenommen wird, durch welchen das Instrument eingeführt wird, so dass es sich wieder im Inneren des Darms befindet. Nach erfolgter Anastomose durch das Klammernahtgerät beziehungsweise TFT-Instrument wird dieses wieder entfernt. Der seitliche Längsschnitt an der einen Darmwand wird manuell vernäht. Ein für diese Vorgehensweise geeignetes chirurgisches System ist beispielsweise aus DE 10 2010 020 664 A1 bekannt.

Ein anderes Verfahren und Instrument ist aus WO 03/061487 A1 und DE 10 2008 048 293 A1 bekannt. Hierbei wird zur Herstellung einer Gefäßanastomose das Ende eines Blutgefäßes durch eine Hülse geführt und derart umgeschlagen, dass das Gewebe auf der Außenseite der Hülse zu liegen kommt. Das Ende des zweiten Blutgefäßes wird dann über das erste Blutgefäß gezogen. Die Hülse ist mit einer ersten Elektrodenanordnung versehen, welche sich am Außenumfang der Hülse befindet. Über die beiden auf die Hülse gezogenen Gefäßabschnitte wird ein Ring gebracht, der an seinem Innenumfang eine zweite umlaufende Elektrodenanordnung aufweist. So können die beiden Gefäßabschnitte zwischen den beiden Elektrodenanordnungen an dem Außenring und der Hülse bei Anlegen eines HF-Stroms miteinander fusioniert werden.

Durch die axiale Überlappung der Gefäßabschnitte kommt es dabei jedoch im Verbindungsbereich zu einer Gewebeverdickung beziehungsweise zu einer Verengung des Innenquerschnitts im Bereich der Verbindungsstelle.

Die US 2007/276363 A1 offenbart ferner ein Instrument und ein Verfahren zur End-zu-End-Wiederverbindung von Darmgewebe. Außerdem ist aus der US 2010/100122 A1 ein Verfahren zum Versiegeln von Gewebe mit Hochfrequenzenergie bekannt. Die WO 99/65409 A1 offenbart nahtfreie Anastomose-Systeme.

Aufgabe der vorliegenden Erfindung ist somit die Bereitstellung eines elektrochirurgischen Instruments zur Herstellung einer End-zu-End-Anastomose, das die eingangs geschilderten Nachteile des Standes der Technik überwindet, für Gefäß- und Darmanastomosen geeignet ist und einen geschmeidigen Übergang an der Verbindungsstelle der beiden zu verbindenden Hohlorganabschnitte schafft.

Diese Aufgabe wird durch ein Anastomose-Instrument mit den Merkmalen des Anspruchs 1 gelöst.

Ein erfindungsgemäßes elektrochirurgisches Instrument weist zwei relativ zueinander bewegbare Werkzeuge auf, die jeweils mit einer Elektrode versehen sind, mit welchen die Hohlorganabschnitte miteinander (thermo-)fusioniert werden können. Die Elektroden können als HF-Elektroden ausgebildet sein. Die beiden Werkzeuge haben im Wesentlichen die Form einer Hülse oder sind zumindest in eine Hülsenform bringbar, so dass eines der beiden Werkzeuge um einen ersten Hohlorganabschnitt und das andere der beiden Werkzeuge um einen zweiten Hohlorganabschnitt geführt werden kann und diesen umschließen kann. Die Elektroden sind jeweils an einer Stirnseite der Werkzeuge ausgebildet und die jeweiligen Hohlorganabschnitte lassen sich um diese Stirnseite beziehungsweise Elektrode herum von innen nach außen umstülpen. Ferner sind die beiden Werkzeuge so zueinander bewegbar, dass die Elektroden miteinander fluchten und dabei die umgestülpten Hohlorganabschnitte zwischen sich einklemmen können.

Das erfindungsgemäße Instrument lässt sich von außen an die beiden zu verbindenden Hohlorganabschnitte führen, so dass im Gegensatz zu den eingangs beschriebenen zirkulären Klammernahtgeräten beziehungsweise TFT-Instrumenten kein Längsschnitt an einem Hohlorganabschnitt zum Einführen des Instruments notwendig ist. Dadurch kann dem Patienten das mit dem Schnitt verbundene zusätzliche Trauma erspart bleiben. Ferner kann die Zeit eingespart werden, welche für das manuelle Vernähen dieses Längsschnitts notwendig wäre. Durch die Verwendung von Elektroden anstatt von Klammern entsteht eine umlaufende geschlossene Fusionsbahn und somit eine sichere Verbindung zwischen den beiden Hohlorganabschnitten. Darüber hinaus kann der Durchmesser der zu verbindenden Hohlorgane relativ klein sein. Schließlich wird ein bündiger Übergang zwischen den Hohlorganabschnitten erreicht, da die Hohlorganabschnitte zwischen zwei Ringelektroden, die an den Stirnseiten der hülsenförmigen Werkzeuge angeordnet sind, eingeklemmt und miteinander fusioniert werden. Dadurch haben die beiden Hohlorganabschnitte im Verbindungsbereich den gleichen Innenquerschnitt, so dass im Verbindungsbereich keinerlei Verengung entsteht.

Vor der Thermofusion der beiden Hohlorganabschnitte müssen die beiden Werkzeuge so an den beiden zu verbindenden Hohlorganabschnitten platziert werden, dass die Werkzeuge den jeweiligen Hohlorganabschnitt umschließen und jeweils ein überstehender Endabschnitt von innen nach außen auf die Außenfläche des jeweiligen Werkzeugs umgestülpt werden kann. Um die Handhabung hierfür zu erleichtern und in axialer Richtung mehr Platz zum Umstülpen der jeweiligen Hohlorganabschnitte zu schaffen, können gemäß einem anderen oder zusätzlichen Aspekt die beiden Werkzeuge an einem Instrumentengrundkörper zueinander verdrehbar gelagert sein. Dadurch lassen sich die beiden Werkzeuge zumindest zwischen einer fluchtenden und einer nichtfluchtenden Stellung verdrehen. In der zueinander verdrehten Stellung behindern die beiden hülsenförmigen Werkzeuge nicht gegenseitig den Umstülpvorgang der Hohlorganabschnitte. Ferner kann bei Verwendung eines Stabilisatorstabes oder Stützdorns zum Aufweiten des jeweiligen Hohlorganabschnitts dieser leichter in axialer Richtung eingeführt und wieder entfernt werden.

Um die Werkzeuge leichter von außen an die jeweiligen Hohlorganabschnitte anzusetzen, können die hülsenförmigen Werkzeuge gemäß einem anderen oder zusätzlichen Aspekt der Erfindung jeweils als ein Paar Halbschalen gebildet ausgebildet sind, welche zu- und auseinander bewegbar sind und in der geschlossenen Stellung den jeweiligen Hohlorganabschnitt zwischen sich aufnehmen und umschließen können. Durch die Längsteilung der hülsenförmigen Werkzeuge müssen die Hohlorganabschnitte nicht durch die Werkzeuge eingefädelt werden, sondern können in der geöffneten Stellung seitlich an den jeweiligen Hohlorganabschnitt herangeführt werden und anschließend geschlossen werden. Aufgrund dieser Zweiteilung der Werkzeuge wird die Ringelektrode durch zwei an den jeweiligen Halbschalen angeordnete halbkreisförmige Elektrodensegmente gebildet, welche in der geschlossenen Stellung eine geschlossene Ringelektrode bzw. ringförmige Elektrodenanordnung bilden.

Gemäß einem anderen oder zusätzlichen Aspekt der Erfindung kann die geschlossene Stellung der Halbschalen arretierbar sein, so dass sich diese nicht versehentlich wieder öffnen. Alternativ oder zusätzlich können die Halbschalen in die geschlossene Stellung zum Beispiel mittels einer Feder vorgespannt sein.

Gemäß einem anderen oder zusätzlichen Aspekt der Erfindung können die beiden Halbschalenpaare an dem Instrumentengrundkörper relativ zu- und auseinander bewegbar und/oder verdrehbar gelagert sein. Dadurch lassen sich die Halbschalen und die Ringelektroden über eine definierte Bewegung in die Thermofusionsstellung bringen und nach der Thermofusion wieder separieren. Dies kann durch eine axiale Verschiebbarkeit der Werkzeuge beziehungsweise der Halbschalen am Grundkörper und/oder eine Verdrehbarkeit in Umfangsrichtung zum Grundkörper erreicht werden.

Gemäß einem Aspekt der Erfindung können die rotatorischen oder translatorischen Freiheitsgrade der einzelnen Halbschalen oder Halbschalenpaare durch Anschläge und/oder Kulissen begrenzt werden. Dies erleichtert wesentlich die Handhabung des elektrochirurgischen Instruments während des operativen Eingriffs, da die Werkzeuge beziehungsweise Elektroden lediglich in geeignete und vorbestimmte Stellungen gebracht werden können.

Gemäß einem anderen oder zusätzlichen Aspekt der Erfindung können die Halbschalenpaare nur in der jeweils geschlossenen und fluchtenden Stellung axial aufeinander zu bewegt werden. Dadurch wird sichergestellt, dass sich die beiden Ringelektroden in einer fluchtenden Stellung einander gegenüber liegen, wenn die Elektroden mit HF-Strom beaufschlagt werden, und nicht versehentlich zueinander verdreht sind, was dazu führen könnte, dass die beiden Hohlorganabschnitte nicht oder nur teilweise miteinander verbunden werden.

Die beiden Halbschalenpaare können gemäß einem anderen oder zusätzlichen Aspekt der Erfindung in der geschlossenen und fluchtenden Stellung über eine Feder mit einer vorbestimmten Kraft gegeneinander vorgespannt sein. Dabei kann es sich um eine am Instrumentengrundkörper abgestützte Druckfeder handeln. Durch die Feder kann die Kraft mit welcher die beiden Elektroden gegeneinander vorgespannt werden und zwischen sich die zu verbindenden Hohlorganabschnitte einklemmen, so eingestellt werden, wie es für die Gewebefusion günstig ist. Somit werden die Hohlorganabschnitte zwischen den Elektroden stets mit derselben Vorspannkraft eingeklemmt, unabhängig von der jeweiligen Bedienperson des elektrochirurgischen Instruments. So wird stets eine zuverlässige Verbindung zwischen den beiden Hohlorganabschnitten erreicht. Anstelle einer planen Ringfläche an den Stirnseiten der Werkzeuge beziehungsweise Halbschalen, kann die Stirnseite des einen Halbschalenpaars eine Innenkonusfläche und die Stirnseite des anderen Halbschalenpaars eine dazu komplementäre Außenkonusfläche aufweisen, welche jeweils mit Elektroden versehen sind, so dass die umgestülpten Hohlorganabschnitte zwischen den an den Konusflächen vorgesehenen Elektroden eingeklemmt werden können. Durch diese besondere Ausgestaltung der Stirnseiten der beiden Werkzeuge des elektrochirurgischen Instruments kann die Verbindungsfläche zwischen den beiden Hohlorganabschnitten vergrößert werden.

Gemäß einem anderen oder zusätzlichen Aspekt der Erfindung können die Innen- und Außenflächen der Halbschalen aus elektrisch nicht leitendem Material ausgebildet sein. Das heißt, die hülsenförmigen Werkzeuge haben einen elektrisch leitenden Kern, der in radialer Richtung, das heißt nach innen und nach außen elektrisch isoliert ist und lediglich an der Stirnseite eine ringförmige Elektrodenfläche aufweist. Dadurch wird sichergestellt, dass der Strom lediglich an der gewünschten Stirnseite mit dem Gewebe in Berührung kommt. Im Übrigen lässt sich eine solche Hülse sehr einfach fertigen.

Die Werkzeuge beziehungsweise die Halbschalen können gemäß einem anderen oder zusätzlichen Aspekt der Erfindung an der Außenfläche hinter den Stirnseiten, welche die Elektroden aufweisen, ein oder mehrere Rückhalteelemente aufweisen, welche den jeweiligen umgestülpten Hohlorganabschnitt fixieren können. Dadurch wird erreicht, dass die beiden Hohlorganabschnitte in der umgestülpten Position verbleiben und nicht nach dem Umstülpen wieder von den Werkzeugen herunter gleiten.

Diese Rückhaltelemente können durch eine Vielzahl an Widerhaken und/oder einer scharfen umlaufenden Kante, Haltedorne etc. Durch die Ausbildung der Rückhalteelemente unmittelbar an den Werkzeugen beziehungsweise Halbschalen können zusätzliche Haltevorrichtungen oder Haltemaßnahmen eingespart werden.

Gemäß einem anderen oder zusätzlichen Aspekt der Erfindung kann zwischen den Rückhalteelementen und der Stirnkante der Werkzeuge eine Ringnut ausgebildet sein, welche zur Führung eines Skalpells beim Abschneiden von überstehendem Gewebe dient. Nach der Gewebefusion der beiden Hohlorganabschnitte an den ringförmigen Stirnseiten der Werkzeuge wird das umgestülpte überstehende Gewebe nicht mehr benötigt. Durch die Ausbildung einer Rille oder Nut an der Werkzeugaußenfläche unmittelbar hinter der Stirnseite beziehungsweise Stirnkante braucht der Chirurg lediglich das Skalpell entlang dieser Ringnut führen, um das überstehende Gewebe sauber abzutrennen. Das abgeschnittene überstehende Gewebe verbleibt an den Rückhalteelementen und kann zusammen mit dem elektrochirurgischen Instrument entfernt werden.

Das elektrochirurgische Instrument kann gemäß einem anderen oder zusätzlichen Aspekt der Erfindung ferner einen an dem Instrument unmittelbar vorgesehenen oder separaten Stabilisatorstab aufweisen, der zum Einführen und Aufweiten des jeweiligen Hohlorganabschnitts dient. Vor dem Umstülpen stützt der Stabilisatorstab den Hohlorganabschnitt an seiner Innenseite, während die Werkzeughalbschalen den Hohlorganabschnitt an seiner Außenseite stützen. Somit ermöglicht bzw. erleichtert der Stabilisatorstab ein definiertes Umstülpen des Hohlorganabschnitts.

Gemäß einer Ausführungsform kann der Stabilisatorstab ein abgerundetes oder konisch ausgebildetes vorderes Ende und einen im Wesentlichen mittig angeordneten Radialflansch mit einer umlaufenden Ausrundung aufweisen. Wenn der Stabilisatorstab in den Hohlorganabschnitt eingeführt wird und der Hohlorganabschnitt auf die Ausrundung trifft, wird der Endabschnitt des Hohlorganabschnitts durch die Ausrundung umgestülpt, ohne dass der Operateur mit der Hand oder anderen Hilfsmitteln wie Pinzetten den Hohlorganabschnitt umstülpen muss. Nach dem Umstülpen des Hohlorganabschnitts auf die Außenfläche des jeweiligen Werkzeugs mit Hilfe des Stabilisatorstabs kann dieser wieder entfernt werden und gegebenenfalls für das Umstülpen des anderen der beiden zu verbindenden Hohlorganabschnitte verwendet werden.

Ein unbeanspruchter Aspekt der Erfindung betrifft den Stabilisatorstab selbst, d.h. ohne das elektrochirurgischen Instrument. Demnach ist ein Stabilisatorstab zum Einführen in und Aufweiten eines Hohlorganabschnitts vorgesehen, wobei der längliche, im Wesentlichen rotationssymmetrische Stabilisatorstab ein abgegrundetes oder konisch ausgebildetes vorderes Ende, einen zylindrischen Abschnitt, dessen Durchmesser im Wesentlichen dem Hohlorganabschnitte entspricht, und einen daran anschließenden, sich radial nach außen erstreckenden Abschnitt aufweist, wobei der sich radial nach außen erstreckenden Abschnitt mit einer umlaufenden, U-förmigen Ausrundung zum Umstülpen des offenen Endes des Hohlorganabschnitts versehen ist.

Der Instrumentengrundkörper, an dem die beiden Werkzeuge angeordnet sind, kann einen HF-Anschluss aufweisen, mit welchem die Elektroden elektrisch verbunden sind. Dieser Anschluss kann ein bipolarer HF-Anschluss sein. Über den HF-Anschluss, kann das elektrochirurgische Instrument mit einem entsprechenden HF-Stromgenerator verbunden werden, der den für die Thermofusion der Hohlorganabschnitte nötigen HF-Strom erzeugt.

Ein beispielhafter sowie unbeanspruchter Aspekt der vorliegenden Erfindung betrifft ein Verfahren zur Herstellung einer End-zu-End-Anastomose zwischen zwei Hohlorganabschnitten, die mit zwei Elektroden miteinander fusioniert werden, wobei die Elektroden an zwei relativ zueinander bewegbaren Werkzeugen eines elektrochirurgischen Instruments vorgesehen sind und die beiden Werkzeuge im Wesentlichen hülsenförmig ausgebildet sind beziehungsweise eine Hülsenform annehmen können und die Elektroden jeweils an einer Stirnseite der Werkzeuge ausgebildet sind. Das Verfahren weist folgende Schritte auf: Umschließen eines ersten Hohlorganabschnitts mit einem ersten Werkzeug; Umschließen eines zweiten Hohlorganabschnitts mit einem zweiten Werkzeug; Umstülpen der Hohlorganabschnitte um die mit Elektroden versehenen Stirnseiten des jeweiligen Werkzeugelements; Zueinanderbewegen der beiden Werkzeuge, so dass die Stirnseiten mit den Elektroden fluchten und die umgestülpten Hohlorganabschnitte dazwischen eingeklemmt werden; und Beaufschlagen der Elektroden mit HF-Strom.

Durch das beispielhafte Verfahren wird eine sichere End-zu-End-Anastomose hergestellt, ohne dabei ein zusätzliches Trauma infolge eines seitlichen Schnitts eines Hohlorganabschnitts herbeizuführen und ohne dabei im Bereich der Verbindungsstelle eine Verdickung beziehungsweise Verengung des Innenquerschnitts im Bereich der Verbindungsstelle herbeizuführen. Durch Verwendung der Gewebefusionstechnik wird zudem eine bessere Verbindung als durch klammern erreicht.

Gemäß einem anderen oder zusätzlichen, beispielhaften Aspekt kann das Verfahren die folgenden Schritte aufweisen: Einführen eines Stabilisatorstabs in den jeweiligen Hohlorganabschnitt; Umschließen des jeweiligen Hohlorganabschnitts mit dem entsprechenden Werkzeugelement; Umstülpen des jeweiligen Hohlorganabschnitts um die mit Elektroden versehene Stirnseite über das entsprechende Werkzeug; und Entfernen des Stabilisatorstabs aus dem jeweiligen Hohlorganabschnitt.

Der dabei verwendete Stabilisatorstab kann die oben erwähnten Merkmale aufweisen. Durch diese beispielhafte Vorgehensweise können die miteinander zu verbindenden Hohlorganabschnitte in die gewünschte, im Wesentlichen zylindrische Form gebracht werden. Wenn die Hohlorganabschnitte über das jeweilige hülsenförmige Werkzeug gestülpt sind, wird die gewünschte Form der Hohlorganabschnitte durch die Werkzeuge definiert bzw. gehalten, so dass der Stabilisatorstab entfernt werden kann.

Gemäß einem anderen oder weiteren beispielhaften Aspekt kann das erfindungsgemäße Verfahren die folgenden Schritte aufweisen: Einführen eines Stabilisatorstabs in den ersten Hohlorganabschnitt; Umschließen des ersten Hohlorganabschnitts mit dem ersten Werkzeug; Umstülpen des ersten Hohlorganabschnitts um die mit Elektroden versehene Stirnseite über das erste Werkzeug; Entfernen des Stabilisatorstabs aus dem ersten Hohlorganabschnitt; Einführen des Stabilisatorstabs in den zweiten Hohlorganabschnitt; Umschließen des zweiten Hohlorganabschnitts mit dem zweiten Werkzeug; Umstülpen des zweiten Hohlorganabschnitts um die mit Elektroden versehene Stirnseite über das zweite Werkzeug; Entfernen des Stabilisatorstabs aus dem zweiten Hohlorganabschnitt. Erfindungsgemäß kann somit ein Stabilisatorstab zum Aufweiten und in Form bringen beider Hohlorganabschnitte verwendet werden und wenn der jeweilige Hohlorganabschnitt durch das entsprechende Werkzeug in seiner Form gehalten wird, entfernt werden.

Gemäß einem anderen oder weiteren beispielhaften Aspekt kann das Verfahren folgende Schritte aufweisen: Beabstanden der beiden Werkzeuge um einen vorbestimmten Abstand; Verdrehen der beiden Werkzeuge um einen vorbestimmten Winkel, insbesondere einem Winkel zwischen 20 und 40°, bevorzugt 30°; Öffnen der Halbschalen der beiden Werkzeuge; Einführen eines Stabilisatorstabs in den ersten Hohlorganabschnitt; Schließen der Halbschalen des ersten Werkzeugs über dem ersten Hohlorganabschnitt, so dass der erste Hohlorganabschnitt am zu verbindenden Ende über die Halbschalen des ersten Werkzeugs übersteht; Umstülpen des ersten Hohlorganabschnitts um die mit Elektroden versehene Stirnseite über das erste Werkzeug; Vorübergehendes Fixieren des umgestülpten ersten Hohlorganabschnitts außen am ersten Werkzeug; Entfernen des Stabilisatorstabs aus dem ersten Hohlorganabschnitt; Einführen des Stabilisatorstabs in den zweiten Hohlorganabschnitt; Schließen der Halbschalen des zweiten Werkzeugs über dem zweiten Hohlorganabschnitt, so dass der zweite Hohlorganschnitt am zu verbindenden Ende über die Halbschalen des zweiten Werkzeugs übersteht; Umstülpen des zweiten Hohlorganabschnitts um die mit Elektroden versehene Stirnseite über das zweite Werkzeug; Vorübergehendes Fixieren des umgestülpten zweiten Hohlorganabschnitts außen am zweiten Werkzeug; Entfernen des Stabilisatorstabs aus dem zweiten Hohlorganabschnitt; Zurückdrehen der beiden Werkzeuge um den vorbestimmten Winkel, so dass die Stirnseiten der beiden Werkzeuge fluchten; Zusammenführen der beiden Werkzeuge und Einklemmen der beiden Hohlorganabschnitte zwischen den Elektroden der jeweiligen Werkzeuge; Beaufschlagen der Elektroden mit HF-Strom; Abtrennen der überstehenden, umgestülpten Gewebeenden der jeweiligen Hohlorganabschnitte; Öffnen der Halbschalen der beiden Werkzeuge; und Entfernen der beiden Werkzeuge von den verbundenen Hohlorganschnitten.

### Kurze Beschreibung der Zeichnungen

Die vorliegende Erfindung wird anhand von beigefügten Zeichnungen näher dargestellt. Es zeigen:
- Figur 1: ein elektrochirurgisches Instrument gemäß einer ersten Ausführungsform der Erfindung;
- Figuren 2A bis 2D: verschiedene Phasen einer End-zu-End-Anastomose mit Hilfe des elektrochirurgischen Instruments der ersten Ausführungsform;
- Figur 3: eine Seitenansicht einer Werkzeughalbschale des elektrochirurgischen Instruments der ersten Ausführungsform;
- Figur 4: eine Teilansicht zweier Werkzeughalbschalen des elektrochirurgischen Instruments der ersten Ausführungsform;
- Figur 5: eine Seitenansicht eines zum elektrochirurgischen Instrument zugehörigen Stabilisator;
- Figur 6: ein elektrochirurgisches Instrument gemäß einer zweiten Ausführungsform der Erfindung; und
- Figur 7: eine Prinzipskizze zweier Werkzeuge eines elektrochirurgischen Instruments gemäß einer dritten Ausführungsform.

### Detailliert Beschreibung von Ausführungsformen

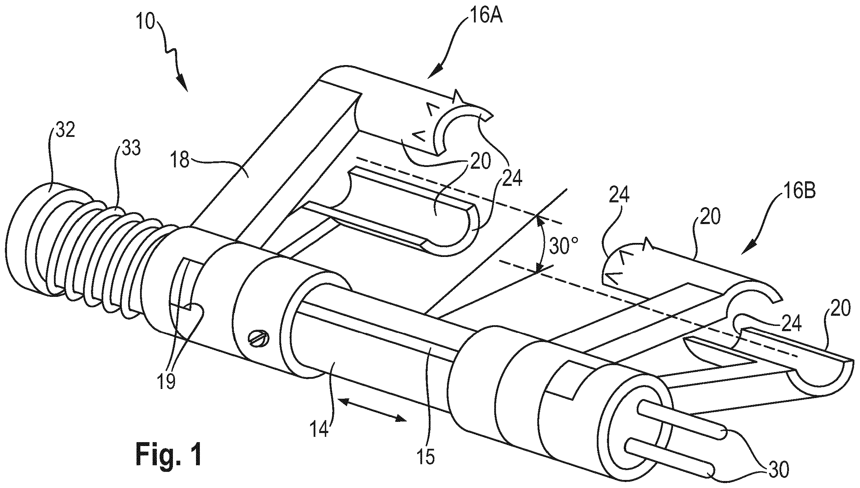

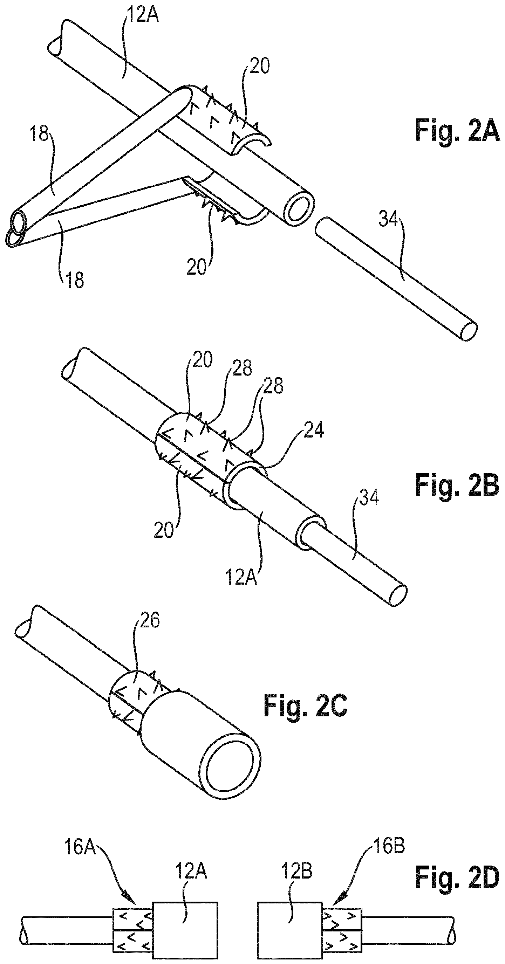

Die Figur 1 zeigt ein elektrochirurgisches Instrument 10, mit welchem eine End-zu-End-Anastomose zwischen zwei Hohlorgan- oder Gefäßabschnitten 12 (siehe Fig. 2) hergestellt werden kann. Das Instrument 10 weist einen länglichen Grundkörper 14 auf, an dem zwei Werkzeuge 16 (16A und 16B) angeordnet sind. Jedes der Werkzeuge 16 ist zweiteilig aufgebaut. Jedes Werkzeugteil weist einen Ausleger 18 auf, welcher mit dem Grundkörper 14 verbunden ist und sich quer zur Längserstreckung des Grundkörpers 14 erstreckt. An distalen Enden der Ausleger 18 ist jeweils quer dazu beziehungsweise parallel zum Grundkörper 14 eine zylindrische Halbschale 20 angeordnet. Die Ausleger 18 sind drehbar am Grundkörper 14 gelagert, so dass zum einen jedes Auslegerpaar 18 um die Längsachse des Grundkörpers 14 gedreht werden kann und auch die Ausleger 18 relativ zueinander gedreht werden können, um die Halbschalen 20 in eine geöffnete und eine geschlossene Stellung zu bringen. Die Halbschalen 20 sind symmetrisch ausgebildet, so dass sich die Halbschalen 20 in der geschlossenen Stellung zu einem Hohlzylinder beziehungsweise einer Hülsenform vervollständigen. Der Ausleger 18 und die Halbschale 20 haben im Wesentlichen eine L-Form, wobei die beiden Werkzeuge 16A und 16B ebenfalls symmetrisch ausgebildet sind und die Halbschalen 20 der beiden Werkzeuge 16A und 16B aufeinander zu weisen.

An den aufeinander zu weisenden Stirnseiten 22 der Halbschalen 20 sind jeweils Elektroden 24 oder eine Anordnung von Elektroden 24 vorgesehen, die in der geschlossenen Stellung der Halbschalen 20 eine umlaufende Elektrodenfläche ausbilden beziehungsweise mehrere umlaufende Elektrodensegmente aufweisen.

Eines oder beide Werkzeuge 16A und 16B lassen sich auf dem Grundkörper 14 axial verschieben und sich somit aufeinander zu- und von einander wegbewegen. Ferner kann eines oder beide Werkzeuge 16 um die Längsachse des Grundkörpers 12 verdreht werden, so dass nicht nur die Werkzeugteile (Ausleger 18 und Halbschalen 20) zueinander verdreht beziehungsweise geschlossen oder geöffnet werden können, sondern auch die Werkzeuge 16 insgesamt zwischen einer fluchtenden und nicht fluchtenden Stellung bringbar sind.

Im Grundkörper 14 sind Kulissen 15 vorgesehen, welche die Drehbewegung und axiale Verschiebung der beiden Werkzeuge 16A und 16B definieren. Die Kulissen 15 erlauben zum einen ein Öffnen der Ausleger 18 bzw. Halbschalen 20 und ein Verdrehen der Werkzeuge 16 zueinander und verhindern zum anderen, dass bei geöffneten Halbschalen 20 und/oder zueinander verdrehten Werkzeugen 16A und 16B eine axiale Annährung der Halbschalen 20 an die gegenüberliegenden Halbschalen 20 stattfindet. Bei geschlossenen Halbschalen 20 und zurückgedrehten bzw. fluchtenden Werkzeugen 16 dagegen wird eine axiale Bewegung zugelassen und sichergestellt, dass die Stirnseiten 22 bzw. Elektroden 24 exakt aufeinander treffen.

Die Ausleger 18 weisen Anschläge 19 auf, welche den maximalen Öffnungsgrad der Ausleger 18 bzw. der Halbschalen 20 begrenzen.

Aus der Figur 1 ist ferner erkennbar, dass unmittelbar hinter der Stirnseite 22, welche mit den Elektroden 24 versehen ist, jede Halbschale 20 an ihrer Außenfläche 26 Rückhaltenasen oder Elemente oder Dorne 28 aufweist, deren Funktion weiter unten detailliert beschrieben wird.

An einem axialen Ende des Grundkörpers 14 ist ein HF-Anschluss 30 vorgesehen, über welchen das elektrochirurgische Instrument 10 mit einer (nicht gezeigten) HF-Stromquelle verbunden werden kann.

Die Elektroden 24 an den Halbschalen 20 sind über den Ausleger 18 und den Grundkörper 14 mit dem HF-Anschluss 30 elektrisch verbunden, so dass die Elektroden 24 mit HF-Strom beaufschlagt werden können. Am anderen axialen Ende ist ein Flansch 32 vorgesehen, an den sich eine Druckfeder 33 abstützen kann, welche das eine Werkzeug 16A in Richtung des anderen Werkzeugs 16B vorspannt. Ferner kann das andere Werkzeug 16B ebenfalls über eine Feder in die andere Richtung gespannt sein.

Die Figuren 2A bis 2D zeigen prinzipiell die Funktionsweise des elektrochirurgischen Instruments 10.

Zunächst wird in den einen Hohlorgan- oder Gefäßabschnitt 12 ein Stabilisatorstab 34 eingeführt, um den Hohlorganabschnitt 12 aufzuweiten bzw. aufzuspannen und in eine im Wesentlichen zylindrische Form zu bringen. Nachdem das zu anastomosierende Gefäß oder Hohlorgang (z.B. Dünn- oder Dickdarm) mit Hilfe des Stabilisators 34 armiert wurde, wird, wie in der Figur 2A gezeigt, das eine Werkzeug 16A in der geöffneten Stellung seitlich an den Hohlorganabschnitt 12 herangeführt, von außen an den Hohlorganabschnitt 12 angesetzt und geschlossen, so dass die beiden Halbschalen 20, wie in der Figur 2b gezeigt, den Hohlorganabschnitt 12 umschließen. Das Werkzeug 16A beziehungsweise die Halbschalen 20 werden dabei so an dem Hohlorganabschnitt 12 angesetzt, dass das offene Ende des Hohlorganabschnitts 12 aus den Halbschalen 20 herausragt. Anschließend wird wie in der Figur 2C gezeigt der vorstehende beziehungsweise herausragende Hohlorganabschnitt 12 um die ringförmigen Elektroden 24 herum umgestülpt, so dass er an der Außenfläche 26 der Halbschalen 20 anliegt. Da wie bereits zuvor erwähnt und in den Figuren 2A bis 2D gezeigt an der Außenfläche 26 der Halbschalen 20 Rückhalteelemente 28 vorgesehen sind, die sich in den umgestülpten Hohlorganabschnitt 12 eindrücken oder einhaken, wird verhindert, dass sich der umgestülpte Hohlorganabschnitt 12 nicht wieder zurück stülpt.

Wenn der überstehende Hohlorganabschnitt 12 wie in der Figur 2C gezeigt umgestülpt ist, kann der Stabilisatorstab 34 entfernt werden. Anschließend können die in den Figuren 2A bis 2C gezeigten Schritte für den anderen Hohlorganabschnitt 12B mit dem anderen Werkzeug 16B durchgeführt werden.

Die Vorbereitung und Fixierung des Gewebes auf den Elektrodenschalen 20 erfolgt relativ einfach, da die Werkzeuge 16A und 16B mit den daran angeordneten Ringelektroden 24 auf dem Grundkörper 14 des Instruments 10 um ca. 30° zueinander verdrehbar sind (siehe Fig. 1).

Nachdem die beiden Gefäß- bzw. Hohlorganabschnitte 12 auf den Elektrodenhalbschalen 20 fixiert worden sind, werden die Werkzeuge 16A und 16B wieder zurückgeschwenkt, so dass die Werkzeuge 16A und 16B bzw. die an den einander gegenüberliegenden Stirnseiten 22 der Halbschalenpaare 20 angeordneten Elektroden 24 fluchten. Die Figur 2D zeigt die beiden zu verbindenden Hohlorganabschnitte 12A und 12B, welche durch die Halbschalenpaare 20 umschlossen werden und deren jeweiligen Endabschnitte jeweils umgestülpt über den Außenseiten 26 der Halbschalenpaare 20 an der Außenseite 26 anliegen und durch die Rückhalteelemente 28 fixiert werden.

Dann können die Werkzeuge 16A und 16B aufeinander zu bewegt werden, dass die beiden umgestülpten Hohlorganabschnitte 12 zwischen den Elektroden 24 eingeklemmt werden. Wenn nun HF-Strom den Elektroden 24 zugeführt wird, werden die beiden Hohlorganabschnitte 12 zwischen den Ringelektroden 24 miteinander fusioniert. Nach der Gewebefusion werden die an der Außenfläche der Halbschalen 18 befindlichen überstehenden Gewebeabschnitte abgeschnitten, die beiden Werkzeuge 16 geöffnet und das Instrument 10 entfernt. Dann sind die beiden Hohlorganabschnitte 12 an einer umlaufenden ringförmigen Fusionsbahn miteinander verbunden.

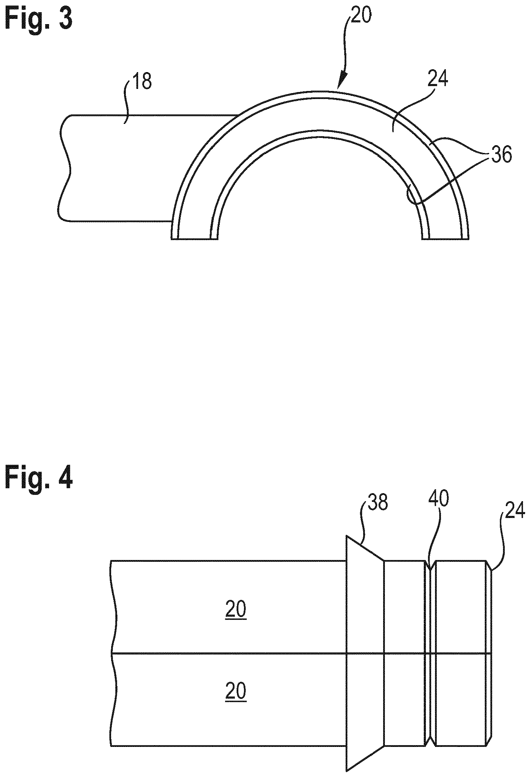

Die Figur 3 zeigt eine Ansicht der Stirnseite 22 einer Halbschale 20, die an dem Ausleger 18 angeordnet ist. Die Stirnseite 22 weist eine halbringförmige Elektrode 24 auf, die sich innerhalb zweier Materialschichten 36 oder Beschichtungen aus elektrisch nicht leitendem Material befindet. Anstelle einer durchgehenden halbringförmigen Elektrode 24, kann auch eine Elektrodenanordnung aus verschiedenen Elektrodensegmenten vorgesehen sein.

Die Figur 4 zeigt eine Seitenansicht zweier Halbschalen 20 in der geschlossenen Stellung, die sich zu einem Hohlprofil beziehungsweise einer Hülse vervollständigen. Anstelle von einer Vielzahl an Dornen 28, wie in den Figuren 1 und 2 gezeigt, ist an der Außenseite 26 der Halbschalen 20 ein umlaufender Rückhaltevorsprung 38 vorgesehen, welcher verhindern soll, dass umgestülptes Gewebe wieder zurück rutscht. Zwischen der Stirnseite 22, welche die Elektroden 24 aufweist, und dem Rückhaltevorsprung 38 ist eine umlaufende Rinne oder Nut 40 vorgesehen, die nach der Gewebefusion als Führungsrinne dient, wenn das überstehende Gewebe abgeschnitten wird, um so sicherzustellen, dass der Chirurg nicht versehentlich mit dem Skalpell die miteinander fusionierten Abschnitte wieder durchtrennt.

Anstelle von mehreren Dornen 28 bzw. umlaufenden Rückhaltevorsprungs 38 können Widerhaken oder andere geeignete Geometrien vorgesehen sein, solange diese verhindern, dass das umgestülpte Gewebe wieder zurück rutscht. Alternativ könnte das Gewebe auch mit anderen separaten Maßnahmen wie einer Klemmhülse oder einer Ligatur mit Faden fixiert werden.

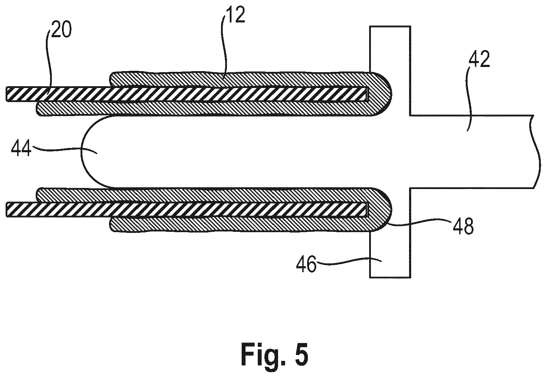

Die Figur 5 zeigt eine alternative Form eines Stabilisatorstabs 42, der neben einer abgerundeten Spitze 44 einen etwas dahinter liegenden umlaufenden Flansch 46 aufweist, der auf der zur Spitze 44 zugewandten Seite eine umlaufende Ausrundung 48 hat, so dass beim Einführen des Stabilisatorstabs 42 in das offene Ende eines Hohlorganabschnitts 12 die Gefäß- beziehungsweise Darmwandung von innen nach außen umgestülpt wird.

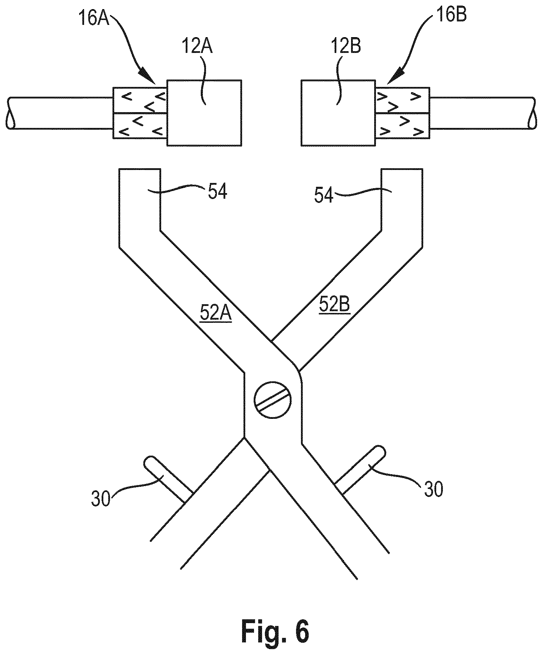

Die Figur 6 zeigt eine zweite Ausführungsform eines elektrochirurgischen Instruments 50, das sich von seiner Funktionsweise von dem Instrument 10 der ersten Ausführungsform nicht unterscheidet und lediglich eine andere Mechanik aufweist, um die beiden Werkzeuge 16A und 16B von einer Vorbereitungsstellung in eine Thermofusionsstellung zu bringen und umgekehrt. Während in der ersten Ausführungsform die Halbschalenpaare 20 über Ausleger 18 an einem länglichen oder stabähnlichen Instrumentengrundkörper 14 axial geführt sind, hat das Instrument 50 im Wesentlichen eine Scheren- beziehungsweise Zangenmechanik 52, an deren distalen Enden 54 die Halbschalen 20 angeordnet sind und über die Scherenmechanik 52 aufeinander zu beziehungsweise auseinander weg bewegt werden können. Die jeweiligen Scherenteile 52A und 52B haben jeweils einen entsprechenden HF-Anschluss zur Stromversorgung von einer HF-Stromquelle. Die Werkzeuge 16A und 16B können mit den Enden 54 der jeweiligen Scherenteile 52A und 52B fest verbunden oder lösbar verbindbar sein. Im letzteren Fall weisen die distalen Enden der Scherenteile 52A und 52B entsprechende Schnappverbindungen oder andere Verbindungsmittel für die Werkzeuge 16 und entsprechende Kontakte auf, um den HF-Strom von den Scherenteilen 52A und 52B zu den Elektroden 24 zuleiten.

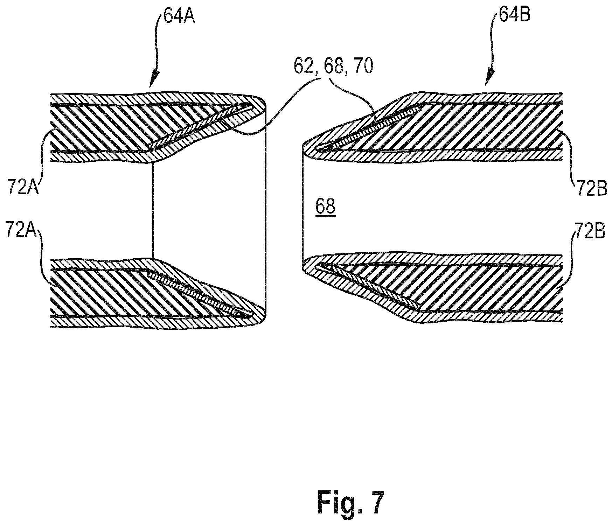

Die Figur 7 zeigt eine dritte Ausführungsform des elektrochirurgischen Instruments 60, das sich von der ersten beziehungsweise zweiten Ausführungsform lediglich in der Gestaltung der Stirnseiten 22 der Halbschalen 20 unterscheiden. Im Gegensatz zur ersten und zweiten Ausführungsform sind die beiden gegenüberliegenden Stirnseiten 62 der Werkzeuge 64 nicht als plane und gleich gestaltete Ringflächen ausgestaltet, sondern weisen einen Innenkonus 66 beziehungsweise einen Außenkonus 68 auf, die komplementär zueinander gestaltet sind. Dabei sind die Elektroden 70 an dem einen Werkzeug 64A beziehungsweise Halbschalenpaar 72A an der Innenkonusfläche 66 ausgebildet und an dem anderen Werkzeug 64B beziehungsweise Halbschalenpaar 72B an der Außenkonusfläche 68 ausgebildet. Wenn die überstehenden Hohlorganabschnitte 12 um diese Elektroden 70 jeweils nach außen umgestülpt werden, und die beiden Werkzeuge 64 so aufeinander zu bewegt werden, dass der Außenkonus 68 des Werkzeugs 64B mit dem Innenkonus 66 des Werkzeugs 16A in Anlage kommt und zwischen sich die umgestülpten Hohlorganabschnitte 12 einklemmen, werden bei Beaufschlagung der Elektroden 70 mit HF-Strom die eingeklemmten Hohlorganabschnitte 12 mit einander fusioniert. Aufgrund der Konusform kann die Breite der Elektroden 70 und somit die Fusionsfläche erhöht werden. Im Übrigen unterscheidet sich die dritte Ausführungsform nicht von der ersten beziehungsweise zweiten Ausführungsform.

Die vorliegende Erfindung ist nicht auf die beschriebenen Ausführungsformen beschrankt. Die Erfindung ist hingegen auf den Schutzumfang des anhängenden unabhängigen Anspruchs 1 beschränkt.

Die Werkzeuge können einteilig, zwei, drei- oder mehrteilig sein, solange sich die Werkzeugelemente bzw. Halbschalen ein geschlossenes Hohlprofil bilden bzw. sich zu einem geschlossenen Hohlprofil zusammenführen lassen und an ihren Stirnseiten eine Ringelektrode oder Ringelektrodenanordnung ausbilden.

Neben den beiden offenbarten Instrumentengrundkörpern sind andere Ausgestaltungen denkbar, solange diese zum einen Öffnen und Schließen der Elektrodenhalbschalen und zum anderen ein Zusammenführen der Stirnseiten der Elektrodenhalbschalen in eine fluchtende Stellung ermöglichen.

Immer wenn in der Beschreibung die Rede von Hohlorgan- oder Darmabschnitten die Rede ist, gilt dies gleichermaßen für Gefäßabschnitte, da das erfindungsgemäße Instrument bei entsprechender Größenanpassung sowohl für Gefäßanastomosen als auch für Darmanastomosen einsetzbar ist.

Anstatt die nach der Gewebefusion verbleibenden, umgestülpten Gewebeabschnitt manuell mit einem Skalpell abzutrennen, kann das Instrument eine Schneideeinrichtung, z.B. eine radial außerhalb der Elektroden vorgesehene, axial verschiebbare Ringschneide, welche nach der Gewebefusion betätigt wird und das Gewebe radial außerhalb der Fusionsbahn abtrennt. Die Ringschneide kann in den Halbschalen integriert sein.

## Patentansprüche

1. Elektrochirurgisches Instrument zur Herstellung (10) einer End-zu-End-Anastomose zwischen zwei Hohlorganabschnitten (12), mit zwei relativ zueinander bewegbaren Werkzeugen (16), die jeweils eine Elektrode (24), vorzugsweise HF-Elektrode, aufweisen, mit welchen die Hohlorganabschnitte (12) miteinander fusioniert werden können, wobei
die beiden Werkzeuge (16) im Wesentlichen hülsenförmig ausgebildet sind oder zumindest eine Hülsenform annehmen können, so dass ein erstes Werkzeug (16A) einen ersten Hohlorganabschnitt (12A) und ein zweites Werkzeug (16B) einen zweiten Hohlorganabschnitt (12B) umschließen kann;
die Elektroden (24) jeweils an einer Stirnseite (22) der hülsenförmigen Werkzeuge (16) ausgebildet sind, um welche herum der jeweilige Hohlorganabschnitt (12) von innen nach außen umstülpbar ist;
die beiden Werkzeuge (16) so zueinander bewegbar sind, dass die Elektroden (24) fluchten und dabei die umgestülpten Hohlorganabschnitte (12A, 12B) zwischen sich einklemmen können; und
die hülsenförmigen Werkzeuge (16) jeweils aus einem Paar Halbschalen (20) gebildet werden, welche zu- und auseinander bewegbar sind, insbesondere zu- und auseinander verschwenkbar sind, und in der geschlossenen Stellung den jeweiligen Hohlorganabschnitt (12) zwischen sich aufnehmen können; wobei die beiden Halbschalenpaare (20) an einem Instrumentengrundkörper (14) axial relativ zu- und auseinander bewegbar und in Umfangsrichtung verdrehbar gelagert sind, **dadurch gekennzeichnet, dass** Kulissen (15) vorgesehen sind, welche die Drehbewegung und axiale Verschiebung der beiden Werkzeuge (16) definieren, und
Anschläge (19) vorgesehen sind, welche den maximalen Öffnungsgrad der Halbschalen (20) begrenzen.

2. Elektrochirurgisches Instrument (12) nach Anspruch 1, wobei die Halbschalenpaare (20) in der geschlossenen und fluchtenden Stellung über eine Feder, insbesondere am Instrumentenkörper (14) abgestützte Druckfeder (33), mit einer vorbestimmten Kraft axial gegeneinander vorgespannt sind.

3. Elektrochirurgisches Instrument (60) nach Anspruch 1 oder 2, wobei die Stirnseite (62) des einen Werkzeugs (64A) eine Innenkonusfläche (66) aufweist und die Stirnseite (62) des anderen Werkzeugs (64B) eine dazu komplementäre Außenkonusfläche (68) aufweist und die umgestülpten Hohlorganabschnitte (12) zwischen den an den Konusflächen (66, 68) vorgesehenen Elektroden (70) eingeklemmt werden können.

4. Elektrochirurgisches Instrument (10) nach einem der Ansprüche 1 bis 3, wobei die Innen- und Außenschichten (36) der Werkzeuge (16) aus elektrisch nicht leitendem Material ausgebildet sind.

5. Elektrochirurgisches Instrument (10) nach einem der Ansprüche 1 bis 4, wobei die Werkzeuge (16) an einer Außenfläche (26) hinter den Stirnseiten (22), welche die Elektroden (24) aufweisen, ein oder mehrere Rückhalteelemente, insbesondere eine Vielzahl an Widerhaken (28) und/oder eine scharfe, umlaufende Kante (38), aufweisen, welche den jeweiligen umgestülpten Hohlorganabschnitt (12) fixieren.

6. Elektrochirurgisches Instrument (10) nach Anspruch 5, wobei zwischen den Rückhalteelementen (28, 38) und der Stirnkante (22) eine Ringnut (40) ausgebildet ist, welche zur Schnittführung beim Abschneiden von überstehendem Gewebe dient.

7. Elektrochirurgisches Instrument (10) nach einem der Ansprüche 1 bis 6, das elektrochirurgische Instrument umfasst ferner einen Stabilisatorstab (42) zum Einführen in und Aufweiten des Hohlorganabschnitts (12), wobei der Stabilisatorstab (42) ein abgegrundetes oder konisch ausgebildetes vorderes Ende (44), einen zylindrischen Abschnitt, dessen Durchmesser im Wesentlichen dem Hohlorganabschnitte (12) entspricht, und einen daran anschließenden, sich radial nach außen erstreckenden Abschnitt (46) aufweist, wobei der sich radial nach außen erstreckenden Abschnitt mit einer umlaufenden, insbesondere U-förmigen, Ausrundung (48) zum Umstülpen des offenen Endes des Hohlorganabschnitts (12) versehen ist.

8. Elektrochirurgisches Instrument (10) nach einem der Ansprüche 1 bis 7, wobei der Instrumentengrundkörper (14) einen, vorzugsweise bipolaren, HF-Anschluss (30) aufweist, mit welchem die HF-Elektroden (24) elektrisch verbunden sind.

## Claims

1. An electrosurgical instrument for making (10) an end-to-end anastomosis between two hollow organ sections (12) comprising two tools (16) movable relative to each other, each including an electrode (24), preferably an HF electrode, by which the hollow organ sections (12) can be fusion-welded to each other, wherein
the two tools (16) are substantially sleeve-shaped or at least can assume a sleeve shape so that a first tool (16A) can enclose a first hollow organ section (12A) and a second tool (16B) can enclose a second hollow organ section (12B),
each of the electrodes (24) is formed at an end face (22) of the sleeve-shaped tools (16) around which the respective hollow organ section (12) can be everted inside out;
the two tools (16) are movable relative to each other so that the electrodes (24) are aligned and can clamp the everted hollow organ sections (12A, 12B) between them; and
each of the sleeve-shaped tools (16) is formed of a pair of half shells (20) which can be moved toward and away from each other, especially can be swiveled toward and away from each other, and in the closed position are adapted to receive the respective hollow organ section (12) between them;
wherein the two pairs of half shells (20) are supported on an instrument body (14) to be axially movable relative toward and away from each other and rotatable in the circumferential direction, **characterized in that**
cranks (15) are provided which define the rotational movement and the axial displacement of the two tools (16), and
stops (19) are provided for limiting the maximum degree of opening of the half shells (20).

2. The electrosurgical instrument (12) according to claim 1, wherein the pairs of half shells (20) are axially prestressed against each other in the closed and aligned position via a spring, especially a compression spring (33) supported on the instrument body (14), by a predetermined force.

3. The electrosurgical instrument (60) according claim 1 or 2, wherein the end face (62) of the one tool (64A) includes an inner conical surface (66) and the end face (62) of the other tool (64B) includes an outer conical surface (68) complementary thereto and the everted hollow organ sections (12) can be clamped between the electrodes (70) provided at the conical surfaces (66, 68).

4. The electrosurgical instrument (10) according to one of the claims 1 to 3, wherein the inner and outer layers (36) of the tools (16) are made of electrically non-conductive material.

5. The electrosurgical instrument (10) according to one of the claims 1 to 4, wherein at an outer face (26) behind the end faces (22) including the electrodes (24), the tools (16) include one or more retaining elements, especially a plurality of barbed hooks (28) and/or a sharp peripheral edge (38) for fixing the respective everted hollow organ section (12).

6. The electrosurgical instrument (10) according to claim 5, wherein between the retaining elements (28, 38) and the front edge (22), an annular groove is formed (40) which serves for guided cutting when projecting tissue is severed.

7. The electrosurgical instrument (10) according to one of the claims 1 to 6, wherein the electrosurgical instrument further comprises a stabilizer rod (42) for being inserted in and expanding the hollow organ section (12), wherein the stabilizer rod (42) includes a rounded or conical front end (44), a cylindrical section whose diameter substantially corresponds to the hollow organ section (12), and a connected portion (46) extending radially outward, wherein the portion extending radially outward is provided with a peripheral, especially U-shaped, rounding (48) for everting the open end of the hollow organ section (12).

8. The electrosurgical instrument (10) according to one of the claims 1 to 7, wherein the instrument body (14) includes a preferably bipolar HF terminal (30) to which the HF electrodes (24) are electrically connected.

## Revendications

1. Instrument électrochirurgical pour la production (10) d'une anastomose bout-à-bout entre deux sections d'organe creux (12), comportant deux outils (16) pouvant être mobiles l'un par rapport à l'autre, qui présentent respectivement une électrode (24), de préférence une électrode HF, avec laquelle les sections d'organes creux (12) peuvent être fusionnées l'une à l'autre, dans lequel
les deux outils (16) sont formés sensiblement en forme de gaine ou peuvent adopter au moins une forme de gaine de sorte qu'un premier outil (16A) peut entourer une première section d'organe creux (12A) et un second outil (16B) peut entourer une seconde section d'organe creux (12B) ;
les électrodes (24) sont formées respectivement sur un côté avant (22) des outils en forme de gaine (16) autour desquels la section d'organe creux respective (12) peut être retournée de l'intérieur vers l'extérieur ;
les deux outils (16) peuvent être mobiles l'un par rapport à l'autre de sorte que les électrodes (24) soient alignées et que les sections d'organes creux ainsi retournées (12A, 12B) puissent se resserrer ; et
les outils en forme de gaine (16) sont formés respectivement à partir d'une paire de demi-coques (20) qui peuvent être rapprochées et écartées, en particulier qui peuvent pivoter en se rapprochant et s'écartant, et dans la position fermée, peuvent recevoir entre elles la section d'organe creux respective (12) ;
dans lequel
les deux paires de demi-coques (20) peuvent se rapprocher et s'écarter relativement l'une de l'autre axialement sur un corps de base d'instrument (14) et sont placées de façon à pouvoir tourner en direction périphérique, **caractérisé en ce que**
des coulisses (15) sont prévues, qui définissent le mouvement de rotation et le décalage axial des deux outils (16), et
des butées (19) sont prévues, qui limitent le degré d'ouverture maximal des demi-coques (20).

2. Instrument électrochirurgical (12) selon la revendication 1, dans lequel les paires de demi-coques (20) sont précontraintes axialement l'une contre l'autre dans la position fermée et alignée sur un ressort, en particulier sur un ressort de compression (33) appuyé sur le corps d'instrument (14).

3. Instrument électrochirurgical (60) selon la revendication 1 ou 2, dans lequel le côté avant (62) de l'un outil (64A) présente une surface conique intérieure (66) et le côté avant (62) de l'autre outil (64B) présente une surface conique extérieure complémentaire (68) et les sections d'organe creux (12) retournées peuvent être serrées entre les électrodes (70) prévues sur les surfaces coniques (66, 68).

4. Instrument électrochirurgical (10) selon l'une quelconque des revendications 1 à 3, dans lequel les couches intérieure et extérieure (36) de l'outil (16) sont formées d'un matériau électriquement non conducteur.

5. Instrument électrochirurgical (10) selon l'une quelconque des revendications 1 à 4, dans lequel les outils (16) présentent sur une surface extérieure (26) à l'arrière des côtés avant (22) qui présentent les électrodes (24), un ou plusieurs éléments de retenue, en particulier une pluralité de barbillons (28) et/ou un bord périphérique tranchant (38) qui fixent la section d'organe creux (12) retournée respective.

6. Instrument électrochirurgical (10) selon la revendication 5, dans lequel entre les éléments de retenue (28, 38) et le bord avant (22) est formée une rainure circulaire (40) qui sert de guidage de coupe lors de la section de tissu dépassant.

7. Instrument électrochirurgical (10) selon l'une quelconque des revendications 1 à 6, l'instrument électrochirurgical comprenant en outre une barre de stabilisation (42) pour l'introduction dans et l'élargissement de la section d'organe creux (12), dans lequel la barre de stabilisation (42) présente une extrémité avant de forme arrondie ou conique (44), une section cylindrique dont le diamètre correspond sensiblement à la section d'organe creux (12), et une section s'y raccordant, s'étendant radialement vers l'extérieur (46), dans lequel la section s'étendant radialement vers l'extérieur est dotée d'un arrondi (48) périphérique, en particulier en forme de U, pour retourner l'extrémité ouverte de la section d'organe creux (12).

8. Instrument électrochirurgical (10) selon l'une quelconque des revendications 1 à 7, dans lequel le corps de base d'instrument (14) présente un raccord HF (30), de préférence bipolaire, avec lequel les électrodes HF (24) sont reliées électriquement.
